# EUROPEAN PATENT SPECIFICATION

(11) **EP 3 354 264 B1**
(45) Date of publication and mention of the grant of the patent: **02.06.2021**
(21) Application number: 18154222.6
(22) Date of filing: 30.01.2018
(51) Int. Cl.: A61K 31/20, A61K 31/201, A61K 31/202, A61K 31/23, A61K 31/231, A61K 31/232, A61K 9/00, A61P 27/02

(54) **PROPHYLAXIS AND TREATMENT OF NEISSERIA GONORRHOEAE EYE INFECTION**
PROPHYLAXE UND BEHANDLUNG EINER AUGENINFEKTION MIT NEISSERIA GONORRHOEAE
PROPHYLAXIE ET TRAITEMENT D'UNE INFECTION OCULAIRE À NEISSERIA GONORRHOEAE

(30) Priority: 30.01.2017 GB 201701450
(43) Date of publication of application: 01.08.2018
(73) Proprietor: Kingston University Higher Education Corporation, Kingston Upon Thames, Surrey KT1 1LQ (GB)
(72) Inventor: SNYDER, Lori A., Kingston upon Thames, Surrey KT1 1LQ (GB); ALANY, Raid G., Kingston upon Thames, Surrey KT1 1LQ (GB)
(74) Representative: Fairbairn, Angus Chisholm

(56) References cited:
- EP-A2- 0 530 861
- WO-A1-2009/072097
- Colin Peter Churchward: "Fatty acids and monoglycerides as novel prophylaxis against gonococcal ophthalmia neonatorum", , November 2016 (2016-11), XP055474307, Retrieved from the Internet: URL:http://eprints.kingston.ac.uk/38330/1/ Churchward-C.pdf [retrieved on 2018-05-11]
- RICHARD D. MILLER ET AL: "Inhibitory Action of Fatty Acids on the Growth of Neisseria gonorrhoeae", INFECTION AND IMMUNITY, vol. 17, no. 2, 1 August 1977 (1977-08-01) , pages 303-312, XP055461506, ISSN: 0019-9567
- H. THORMAR ET AL: "Hydrogels containing monocaprin have potent microbicidal activities against sexually transmitted viruses and bacteria in vitro", SEXUALLY TRANSMITTED INFECTIONS, vol. 75, no. 3, 1 June 1999 (1999-06-01), pages 181-185, XP055474469, GB ISSN: 1368-4973, DOI: 10.1136/sti.75.3.181
- GUDMUNDUR BERGSSON ET AL: "In Vitro Susceptibilities of Neisseria gonorrhoeae to Fatty Acids and Monoglycerides", ANTIMICROBIAL AGENTS AND CHEMOTHERAPY, AMERICAN SOCIETY FOR MICROBIOLOGY, vol. 43, no. 11, 1 November 1999 (1999-11-01), pages 2790-2792, XP002477706, ISSN: 0066-4804
- BUTT UMMARA ET AL: "Fatty acid microemulsion for the treatment of neonatal conjunctivitis: quantification, characterisation and evaluation of antimicrobial activity", DRUG DELIVERY AND TRANSLATIONAL RESEARCH, SPRINGER, GERMANY, vol. 6, no. 6, 20 October 2016 (2016-10-20), pages 722-734, XP036092779, ISSN: 2190-393X, DOI: 10.1007/S13346-016-0338-3 [retrieved on 2016-10-20]
- KRISTMUNDSDOTTIR T ET AL: "Development and evaluation of microbial hydrogels containing monoglyceride as the active ingredient", JOURNAL OF PHARMACEUTICAL SCIENCES, AMERICAN CHEMICAL SOCIETY AND AMERICAN PHARMACEUTICAL ASSOCIATION, US, vol. 88, no. 10, 1 October 1999 (1999-10-01), pages 1011-1015, XP002379551, ISSN: 0022-3549, DOI: 10.1021/JS9900396
- H. GALBRAITH ET AL: "Antibacterial Activity of Long Chain Fatty Acids and the Reversal with Calcium, Magnesium, Ergocalciferol and Cholesterol", JOURNAL OF APPLIED BACTERIOLOGY, vol. 34, no. 4, 1 December 1971 (1971-12-01), pages 803-813, XP055474749, GB ISSN: 0021-8847, DOI: 10.1111/j.1365-2672.1971.tb01019.x
- RASHID S ET AL: "Topical omega 3 and omega 6 fatty acids for treatment of dry eye", ARCHIVES OF OPHTHALMOLOGY, AMERICAN MEDICAL ASSOCIATION, UNITED STATES, vol. 126, no. 2, 1 January 2008 (2008-01-01), pages 219-225, XP002669468, ISSN: 0003-9950, DOI: 10.1001/ARCHOPHTHALMOL.2007.61
- Mary Ann Liebert ET AL: "Final Report on the Safety Assessment of Oleic Acid, Laurie Acid, Palmitic Acid, Myristic Acid, and Stearic Acid", , 1 January 1987 (1987-01-01), XP55648389, Retrieved from the Internet: URL:https://www.cir-safety.org/sites/defau lt/files/115_draft_steary_suppl3.pdf [retrieved on 2019-12-02]

## Description

This invention relates to a novel prophylaxis or treatment against *Neisseria gonorrhoeae* infection.

When an infant is born to a mother infected with the sexually transmitted disease gonorrhoea, caused by the bacteria *Neisseria gonorrhoeae,* up to 48% will develop ophthalmia neonatorum and up to 10% of those who receive prophylaxis will still develop infection. The bacteria cause ulceration of the cornea, perforation, and inflammation (Int.Ophthalmol. 35:135, 2015; BMJ.Clin.Evid. Systematic review 704, 2012). This infection can lead to significant visual loss or blindness, even with treatment. Up to 80% of infected women do not know that they are infected, as they may not have symptoms. Indeed more than 50% of women visiting genitourinary medicine clinics, seeking treatment for a reproductive health concern, had no symptoms of their infection (Sex.Transm.Infect. 85:317, 2009). This means that women are at risk of having an undiagnosed gonorrhoea infection while they are pregnant and passing that infection on to their child during childbirth.

Conjunctivitis that occurs within the first 28 days of life is referred to as ophthalmia neonatorum or neonatal conjunctivitis (J.Antimicrob.Chemother. 14:209, 1984) and includes eye lid swelling and inflammation in the newborn child as well as purulent yellowish discharge from the closed eye. Often the infection will start in one eye and spread across to the other eye during the course of the infection. The primary way in which infants become infected is through transfer of an infectious agent, such as *Neisseria gonorrhoeae* bacteria, from the mother to the eyes of the child during the birthing process. The two most common causes of ophthalmia neonatorum are *Neisseria gonorrhoeae* and *Chlamydia trachomatis,* with the former having the faster onset and usually being more aggressive (J.Midwif.& Womens Health 55: 319, 2010). If *Neisseria gonorrhoeae* ophthalmia neonatorum is not treated, or is unsuccessfully treated, it can cause corneal scarring or corneal perforation which can lead to permanent blindness (Fanaroff and Martin's Neonatal-Perinatal Medicine: Diseases of the Fetus and Infant. Elsevier Health Sciences. pp. 737). The infection can spread from the eyes and cause septicaemia and meningitis, which are life threatening, and arthritis (Int.J.Infect.Dis. 5:139-43, 2001). Infants can also become infected *in utero;* even infants born by Caesarean section are at risk (Sex.Transm.Dis. 6:77, 1979).

The eye infection ophthalmia neonatorum, which can cause permanent blindness, would normally be treated by antibiotics. Prophylaxis currently includes the application of 1 % tetracycline or 0.5 % erythromycin ophthalmic ointment shortly after birth, however, the use of tetracycline-based ophthalmic ointments are in decline due to concerns over antibacterial resistance. Historically, 1-2 % aqueous silver nitrate was used, but there was a very high incidence of treatment-induced conjunctivitis (Int.J.Infect.Dis. 5:139, 2001) and chances of toxicity (Bull.World Health Organ. 67:471, 1989) that were considered to be unacceptable and medically questionable. No prophylactic treatments, including silver nitrate and antibiotics, are optimal in risk-benefit analysis and none are recommended for widespread use (J.Matern.Fetal Neonatal Med. 24:769, 2011). Further, prophylaxis is only 80-90% effective (N.Engl.J.Med. 318:653, 1988 & Bull.World Health Organ. 67:471, 1989), requiring treatment with antibiotics, often ceftriaxone (Antimicrob.Agents Chemother. 43:2790, 1999). For many multidrug-resistant N. *gonorrhoeae,* this is the last antibiotic available and recently there have been completely resistant cases (Antimicrob.Agents Chemother. 55:3538, 2011 & Emerg.Infect.Dis. 17:148, 2011), necessitating a switch to dual therapy because one antibiotic alone is no longer effective. Concern remains about how to properly implement a prophylactic treatment or to choose the correct treatment especially as antimicrobial resistance, including multidrug resistance (Curr.Opin.Infect.Dis. 27:282, 2014), is rising.

In the United Kingdom there has been no prophylaxis since the 1950s and currently only prenatal screening via assessment of risk factors is used (Bull.World Health Organ. 79:262, 2001). Efforts to reduce transmission of gonorrhoea infections from mother to child often include pre-natal screening, but this relies upon several factors in an ideal system. As cost saving measures, not all hospitals and health authorities will screen all pregnant women under their care, choosing instead to only screen those whom they feel are most 'at risk' (Nurs.Womens Health 18:67,2014). In a study looking at compliance with standards of sexually transmitted infection testing during pregnancy, more than 40% of pregnant women who should have been evaluated were not tested at all during pregnancy for gonorrhoea. Since a woman is up to 80% likely to be asymptomatic and a man can be up to 20% asymptomatic as well, it may be that the pregnant woman is not aware of her true risk factors. This is particularly true if her partner is engaging in high risk sexual activities about which she is unaware. This could mean, as well, that an earlier screen could come up as negative, but that the woman could become positive for N. *gonorrhoeae* later in the pregnancy. Indeed, one study explored the risk perception of the sexual health of the male by including screening of male partners in the pre-natal screening programmes, yielding quite interesting reactions from some participants who had not previously considered their role in potential infection transmission to their unborn children (Midwifery 29:351, 2013).

As an additional complication in reducing transmission of gonorrhoea infection through screening mums-to-be, not all women are part of pre-natal screening programmes. A survey of international screening guidelines has reviewed the practices from four countries, including five general antenatal clinical practice guidelines and 20 disease-specific guidelines; the approaches to pre-natal care are vastly different (Int.J.Based Healthc. 12:50, 2014). The UK NICE Clinical Guidelines (http://www.nice.org.uk/guidance/cg62/documents/antenatal-care-full-guideline2) give no recommendations for gonorrhoea and the UK Royal College of Obstetricians and Gynaecologists give no guidelines for gonorrhoea (Int.J.Based Healthc. 12:50, 2014). Even in areas where pre-natal screening for gonorrhoea is typical for all pregnant women, for some women their first contact with the medical system, for one reason or another, is at the delivery of the child. Compliance with pre-natal care is not mandatory and is a complex and often personal situation to unravel.

While it has been typical to discuss infant cases of gonococcal keratitis, adults are also susceptible. An increasing number of reports of adult eye infections are appearing in the literature due to the multi-drug antibiotic resistance state of N. *gonorrhoeae.* Whereas previously a broad spectrum antibiotic would have successfully treated an adult eye infection before it was identified by culture as gonococcus, now such treatments fail, as in a case of a 41 year-old Malay male: even after treatment with intravenous ceftriaxone and hourly ceftazidime drops, the patient's best corrected visual acuity seven months after treatment was 6/9+3 OD and 6/24 OS and his refractive correction was +1.25/-1.50x160 OD and plano OS. The authors of this case note that refraction had been difficult to obtain due to scarring that encroached the visual axis of the left eye (Med.J.Malaysia 61:366, 2006). A paper from 2015 describes a case of a 39 year-old man where a four day course of chloramphenicol had failed to treat an eye infection. When he presented to an eye casualty department, his right eye could only detect hand movements, the upper and lower lids were swollen, there was purulent material in and around the eye, corneal melt had occurred, and the cornea had perforated. Although the man's infection was identified as N. *gonorrhoeae* and he was given intravenous ciprofloxacin and ceftriaxone and hourly topical ofloxacin and erythromycin, he still required multiple grafts to repair the damage to the cornea. Two months after these operations, his best visual acuity was 6/36 on a Snellen chart (Int.Ophthalmol. 35:135, 2015). Another case from the literature presents a very similar situation: a man of 25 years, who presented to an emergency department after three days of experiencing discharge and was given an unsuccessful treatment, so returned in seven days with a very advanced case. His eye was covered in purulent discharge, had corneal melt, and suffered from perforation that ultimately required grafting. His visual acuity, unaided, once the surgery had resolved ten months later, was 6/18 (Sex.Transm.Infect. 86:447, 2010). These authors called for specific UK guidelines in the management of this disease, especially in the face of growing problems of antibiotic resistance; this isolate was resistant to ciprofloxacin and nalidixic acid.

The molecular and genetic mechanisms that underlie the pathogenesis of gonococcal eye infections are poorly understood. A great deal of the focus of gonococcal research is on the sexually transmitted infection and its impact upon reproductive health. With the rise in multi-drug antibiotic resistance, we are facing a future of untreatable gonorrhoea (Curr.Opin.Infect.Dis. 27:282, 2014). In 1880, the pre-antibiotic era, up to 79% of children in institutions for the blind had been blind since birth due to gonococcal ophthalmia neonatorum (Bull.World Health Organ. 67:471, 1989). Without effective treatment infants and adults face a future similar to the pre-antibiotic past.

Diagnoses of cases of gonorrhoea have doubled between 2008 and 2013, with 29,291 new cases in 2013 in England (Public Health England, 2014). Over the years, *Neisseria gonorrhoeae* has developed resistance to the antibiotics used against it, such that now no single antibiotic is recommended. Instead, third generation cephalosporins, the former recommended antimicrobials of choice, are used in combination with azithromycin, because in 2011 the first resistant isolates were reported in Japan (Curr.Opin.Infect.Dis. 27:282, 2014; Antimicrob.Agents Chemother. 55:3538, 2011). Since then, resistant isolates have been reported in North America and Europe(Curr.Opin.Infect.Dis. 27:62,2014; Eurosurveillance 16: 14, 2011; The Lancet Infect.Dis. 14: 220, 2014). The rise in antimicrobial resistance in this species necessitates alternative treatment options.

Every year since the introduction of penicillin, N. *gonorrhoeae* has found a way around multiple attempts to treat it (Clin.Microbiol.Rev. 27:587, 2014). Humans are not able to mount an immunological memory against these bacteria; patients who are successfully treated for the sexually transmitted infection can become re-infected with the same strain from untreated partners (J.Immunol. 188:4008, 2012). There is no vaccine against N. *gonorrhoeae.*

To prevent and/or treat ophthalmia neonatorum, intervention within the first few hours after birth of neonates can prevent infections from establishing. Once an infection is established, as is often the case in adults, it is necessary to treat infections to prevent them from causing blindness and/or damage to the eye.

Postnatal prophylaxis and/or treatment can be in the form of an eye drop, eye ointment, hydrogel, or other appropriate delivery method. The United States Preventive Services Task Force ranks ophthalmia neonatorum prophylaxis "Grade A", which means the net benefit is substantial, and recommends that all newborns be given prophylaxis within 24 hours after birth (Final Evidence Review: Ocular Prophylaxis for Gonococcal Ophthalmia Neonatorum: Preventive Medication. U.S. Preventive Services Task Force, 2012).

The antimicrobial properties of certain fatty acids and fatty acid derivatives have been known for some time (reviewed in Recent Pat.Antiinfect.Drug Discov. 7:111, 2012 & Appl.Microbiol.Biotechnol. 85:1629, 2010). Research on the antimicrobial effects of fatty acids on N. *gonorrhoeae* has been published (Antimicrob.Agents Chemother. 43:2790, 1999). There is also evidence of anti-gonorrhoea activity from several folk remedies containing natural products. Oral administration of a preparation of *Termitomyces microcarpus* (a mushroom) mixed with the pulp of the *Cucurbita pepo* squash and the leaves of *Senna alata* candle bush (Evidence-Based Compl.Alt.Med. 2013:620451, 2013) is reported to cure gonorrhoea sexually transmitted infections (STIs) in Nigeria and other parts of Africa. *Gallesia gorazema* leaves are used as a remedy against gonorrhoea STIs in many areas of Brazil (J.Ethnopharmacol. 150:595, 2013), which is supported by reports from other ethno-medicinal studies of plants being used for the treatment of gonorrhoea in Paraná, Brazil (J.Ethnopharmacol. 161:1, 2015). One fraction extracted from the *G. gorazema* leaves was shown to contain tetradecanal (a reduced form of the fatty acid myristic acid), palmitic acid (a fatty acid), octadecanal (a long-chain aldehyde), γ-sitosterol (similar to cholesterol), β-amyrin and α-amyrin (often found in plants), and three major components that could not be identified (J.Ethnopharmacol. 150:595, 2013). The *Azadirachta indica,* known as neem, is also used to treat gonorrhoea STIs (Int.J.Antimicrob. Agents. 33:86, 2009; J.Biol.Sci. 14:110, 2014) and has been demonstrated to contain fatty acids including stearic acid, oleic acid, and linoleic acid in high percentages (Biochem.Soc.Trans. 28:800, 2000). The mode of action of the antibacterial properties of fatty acids is believed to be action upon the bacterial cell membrane and/or membrane associated proteins and/or components (Recent Pat.Antiinfect.Drug Discov. 7:111, 2012). WO2009/072097A1 and EP0530861A2 describe formulations using combinations of two or more fatty acids and/or derivatives, but do not demonstrate their activity against N. *gonorrhoeae,* or effectiveness of these formulations in the eye. Despite the anti-gonococcal effects of fatty acids and their derivatives being known for 40 years (Infect. Immun. 17(2): 303-312, 1977), an effective use of fatty acids or their derivatives to treat eye infections has not been suggested or contemplated in the literature.

There is a clear need to identify additional treatments against N. *gonorrhoeae* infection of the eye. The present invention proposes to overcome this with a prophylaxis and/or treatment that is not based on antibiotics. It has been known that N. *gonorrhoeae* is resistant to some fatty acids and that fatty acids do not tend to be antimicrobial against Gram negative bacteria; such knowledge may have contributed to the lack of investigation into use of fatty acids as antigonococcal agents. Some fatty acids are substrates of the efflux pump systems that confer resistance to antibiotics, fatty acids, detergents, bile salts, and antimicrobial peptides (J.Mol.Microbiol.Biotechnol. 3:219, 2001). However, this repertoire does not encompass all fatty acids. By taking a multi-disciplinary approach, the inventors have been able to address the World Health Organisation concern about untreatable gonorrhoea.

The present invention offers an alternative to current prophylaxes and treatments in the face of increasing antibiotic resistance, by providing an ocular prophylaxis or ocular treatment for the prevention of ophthalmia neonatorum based on a fatty acid or a fatty acid derivative.

Accordingly, in one aspect, the present invention provides a compound that is a fatty acid or an ester of a fatty acid, or a pharmaceutically acceptable salt or solvate thereof, for use in the ocular treatment or prophylaxis of an infection of the eye caused by *Neisseria gonorrhoeae* in a warm-blooded animal.

In particular, the fatty acid compound is selected from myristoleic acid C14:1cA9, palmitoleic acid C16:1cA9, and capric acid C10:0 monoglyceride (monocaprin C10).

As used herein, the term "fatty acid compound" is used collectively to refer to fatty acids and esters of fatty acids, or their pharmaceutically acceptable salts.

In a preferred embodiment, the fatty acid compound is myristoleic acid C14:1cA9, or capric acid C10:0 monoglyceride (monocaprin C10).

In a most preferred embodiment, the fatty acid compound is capric acid C10:0 monoglyceride (monocaprin C10).

In a further preferred embodiment, the fatty acid compound is in an ophthalmic formulation containing a chelating agent, preferably a calcium ion (Ca²⁺) chelating agent.

In a further preferred embodiment, the use is for an eye infection caused by *Neisseria gonorrhoeae* in a human.

In a further preferred embodiment, the use is for an eye infection caused by *Neisseria gonorrhoeae* in a newborn.

In a further aspect, the present invention provides an ophthalmic formulation comprising a fatty acid compound as defined above, and a pharmaceutically acceptable carrier, for use in the ocular treatment or prophylaxis of an infection of the eye caused by *Neisseria gonorrhoeae.*

In a preferred embodiment, the formulation is in the form of an eye drop liquid, an ointment or a hydrogel.

In a further embodiment, the formulation further comprises a chelating agent.

The fatty acid compound may be formulated in any suitable form for administration to the eye, together with suitable known pharmaceutically acceptable carriers, additives and/or excipients, for example as a liquid for eye drops, an ointment, a hydrogel, or in any other suitable form for application to the eye. Examples of suitable carriers, additives and/or excipients are known in the art. For example, for eye drops or a hydrogel, these may include one or more of a carrier or vehicle (commonly water), a buffering agent (to maintain pH), a tenacity modifier (such as sodium chloride and dextrose), a viscosity modifier (such as cellulose derivatives, dextran, polyvinyl alcohol), and a preservative. For example, for an eye ointment, these may include one or more of a hydrocarbon (such as petrolatum, lanolin, vasoline), and a dispersant and/or surfactant at a low concentration.

In a preferred embodiment, a thickened eye drop would contain the fatty acid compound (such as monocaprin), a non-ionic surfactant (such as polysorbate 20), a solvent (such as propan-1,2-diol), and a polymer (such as HPMC or CMC).

In a preferred embodiment, the formulation is a thickened eye drop, containing HPMC (hydroxypropyl methylcellulose), CMC (carboxymethyl cellulose), or another suitable cellulose- or polymer-based thickening agent. Such components of ocular formulations contribute to stability and solubility.

In a preferred embodiment, the formulation contains an emulsifier such as polysorbate 20, Tween 20. For example, for use of 0.068% to 0.125% monocaprin, a concentration of 0.2% polysorbate 20 would be used, and for 0.188% to 0.25% monocaprin, a concentration of 0.5% polysorbate 20.

In a preferred embodiment, the formulation contains the solvent propane-1,2-diol.

Formulations as described herein have been demonstrated to retain antigonococcal activity for at least 9 months after creation, when stored at room temperature.

If necessary or desired, the fatty acid compound may be used or formulated together with a chelating agent, for example a calcium (Ca²⁺) ion chelating agent. A chelating agent may be included, for example, in order to reduce or prevent any inhibition, if any, of the antibacterial activity of the fatty acid compound caused by calcium (Ca²⁺) ions or other metal ions present in natural tear fluid. Examples of suitable chelating agents which can be used include ethylenediaminetetraacetic acid (EDTA), diethylenetriaminepentaacetic acid (DTPA), edetate calcium disodium hydrate, edetate disodium dihydrate, deferoxamine and penicillamine, in particular EDTA. Examples of other means of protecting the compounds from inhibition include chemical modification of the fatty acids, such as esterification of the fatty acids, in order to render the fatty acids less susceptible to the ionic strength of tear fluid.

Ophthalmia neonatorum, infections of infants shortly after birth, is the most common eye infection caused by N. *gonorrhoeae,* however prophylaxis or potentially treatment of eye infections of humans at any age are within the scope of this invention.

The present invention is useful to target eye infections caused by *Neisseria gonorrhoeae,* but may be effective against other eye infections as well. In particular, monocaprin at the concentration used for the antigonococcal ocular formulation is bactericidal to *Neisseria meningitidis, Staphylococcus aureus, Pseudomonas aeruginosa,* and *Streptococcus pneumoniae* (M. Christodoulides, personal communication).

One or more of the fatty acid compounds may be included in the formulations. Additionally, one or more other active ingredients may be included in the formulations.

The dosage, active ingredients included in the formulations, and method of delivery of the active ingredients may be adjusted, depending upon the treatment needs. Epidemiology may suggest, over time, that one or more of the treatments may prove to be more effective than others and this may vary by country or region. A suitable dosage range for the fatty acid compounds in the eye formulation is from 0.07 mM to 10 mM, preferably from 0.125 mM to 5 mM, in particular from 0.25 mM to 4 mM.

In a preferred embodiment, the fatty acid compound has been solubilised in a solvent, such as propan-1,2-diol, with a non-ionic surfactant, such as polysorbate 20, and with a polymer, such as HPMC or CMC, in a thickened eye drop; for example, a preferred formulation for ocular administration would be 1% HPMC, 0.188% monocaprin, 0.5% polysorbate 20, in which the monocaprin is solubilized in propan-1,2-diol.

The invention has demonstrated bacteriostatic properties, that is, an ability to reduce or prevent growth of N. *gonorrhoeae,* as well as bactericidal properties, that is, an ability to kill N. *gonorrhoeae.* At a sufficient concentration, the compounds are able to both prevent growth of the bacteria and reduce the number of viable bacteria. Moreover, the invention has demonstrated that fatty acid compounds are advantageously non-irritant to the eye, and therefore suitable for ocular administration.

The compounds may be administered in the form of a prodrug which is broken down in the human or animal body to give the parent compound. Prodrugs would be processed by either the host or the bacteria to generate the active ingredient fatty acids or their derivatives. In the present invention, a prodrug may selected that will be broken down on the surface of the eye such that it releases one of the active antimicrobial fatty acid compounds.

Lauric acid (C12:0) is also known as dodecanoic acid, n-dodecanoic acid, dodecylic acid, dodecoic acid, laurostearic acid, vulvic acid, 1-undecanecarboxylic acid, and duodecylic acid.

Tridecanoic acid (C13:0) is also known as tridecylic acid.

Myristoleic acid (C14:1cA9) is also known as (Z)-tetradec-9-enoic acid, 9-tetradecenoic acid, 9-cis-tetradecenoic acid, cis-Δ9-tetradecenoic acid, myristolenic acid, and oleomyristic acid.

Palmitoleic acid (C16:1cA9) is also known as (9Z)-hexadec-9-enoic acid, cis-palmitoleic acid, and 9-cis-hexadecenoic acid.

Linolenic acid (C18:3cA9,12,15) herein refers to α-linolenic acid.

Monocaprin (C10) is a monoglyceride of capric acid (C10:0). Capric acid is also known as decanoic acid, n-capric acid, n-decanoic acid, decylic acid, and n-decylic acid.

Sodium dodecanoate is also known as dodecanoic acid sodium salt, lauric acid sodium salt, and sodium laurate.

Preferred as fatty acid compound for use in accordance with the invention is myristoleic acid C14:1cA9, palmitoleic acid C16:1cA9, or capric acid C10:0 monoglyceride (monocaprin C10). Most preferred as the fatty acid compound is capric acid C10:0 monoglyceride (monocaprin C10).

The invention will now be described solely by way of example and with reference to the accompanying drawings in which it is demonstrated that fatty acid compounds are effective and useful in accordance with the invention for reducing or preventing growth and/or for killing of *N*. *gonorrhoeae,* are non-irritating to the eye, and are active when formulated in ophthalmic drug delivery formulations.

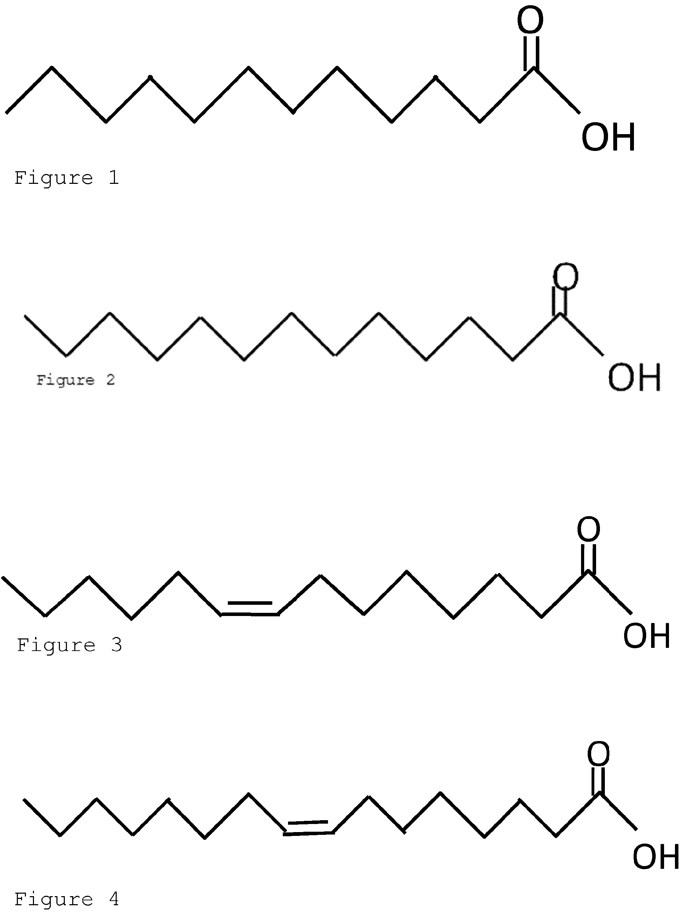

Figure 1 depicts the structure of lauric acid (C12:0).
Figure 2 depicts the structure of tridecanoic acid (C13:0).
Figure 3 depicts the structure of myristoleic acid (C14:1cΔ9).
Figure 4 depicts the structure of palmitoleic acid (C16:1cΔ9).

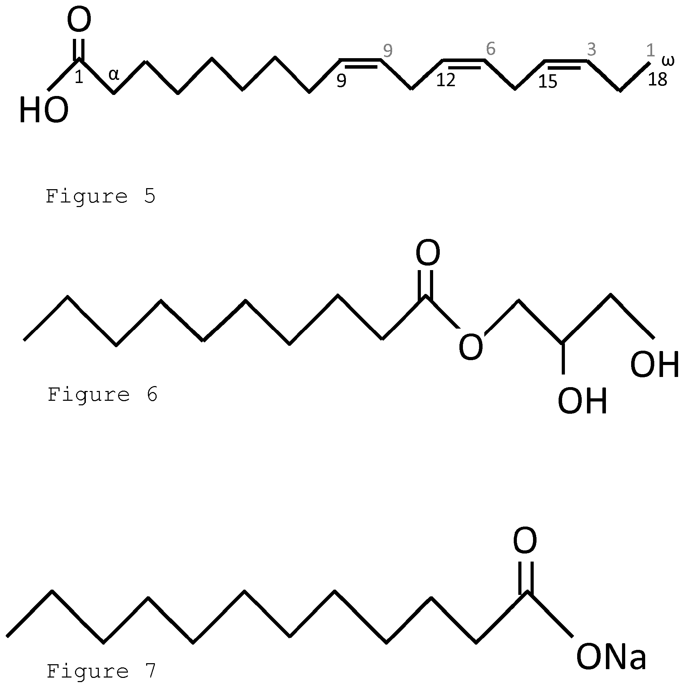

Figure 5 depicts the structure of linolenic acid (C18:3cΔ9,12,15).
Figure 6 depicts the structure of monocaprin (C10).
Figure 7 depicts the structure of sodium dodecanoate.
Figure 8 shows a graph comparing anti-gonococcal activity against N. *gonorrhoeae,* of fatty acid compounds formulated in artificial tear fluid that includes calcium chloride.
Figure 9 shows a graph comparing anti-gonococcal activity against N. *gonorrhoeae,* of fatty acid compounds formulated in artificial tear fluid without calcium chloride.
Figure 10 shows a graph of the log reduction of N. *gonorrhoeae* attached to human cornea epithelial cells when monocaprin (C10) was added immediately after the bacteria or 90 minutes after infection. Means plotted and error bars represent standard deviations (n=3).

### EXAMPLES

### Example 1

### Bacterial strains and culture conditions

The well characterised N. *gonorrhoeae* strain NCCP11945 (Chung GT et al. (2008) Complete genome sequence of Neisseria gonorrhoeae NCCP11945. Journal of Bacteriology 190: 6035-6036) was used in the screening stage of this experiment. Eight isolates that were obtained from Public Health England from various European locations were used in the log reduction assays. All isolates were first cultured for 24 hours prior to testing by plating on GC solid medium (Oxoid Limited, Hampshire, UK) with Kellogg supplements and incubating at 37 °C, 5% CO2 (Kellogg DS, Jr., et al., (1963), J Bacteriol 85: 1274-1279).

### Chemicals

A full list of all the organic acids tested in this study is in Table 1. The tested compounds include saturated and unsaturated fatty acids, monoglycerides, dicarboxylic acids, and fatty acid sodium salts. All compounds investigated were resuspended at a concentration of 100 mM in ethanol unless stated. All chemicals were obtained from Sigma-Aldrich (Poole, Dorset, UK).

### Measurement of bactericidal action of selected fatty acids

Log reductions were done using the same method as in Bergsson et al., (1999), Antimicrob Agents Chemother 43: 2790-2792. Briefly, cells were suspended in GC broth (18 g/L GC Base (Oxiod, Limited) with added defined supplements) to an optical density of 0.3 at 520 nm which is approximately equivalent to 107 - 108 cells per millilitre (Macfarlane DE et al. (1980) Improved media for the culture of Neisseria gonorrhoeae. J Med Microbiol 13: 597-607). Five hundred microliters of the suspension was then added to 5 µl of the 100 mM stocks to give a final concentration of 1 mM. A negative control was also done to calculate the reduction in bacterial titre by adding 500 µl to 5 µl ethanol. The tubes were then mixed at room temperature for two minutes and immediately diluted in a 10 fold dilution series down to 10-4. The dilutions were plated by spreading 10 µl on a quarter of a plate and 100 µl of the neat solution was also plated. Plates were incubated at 37 °C for 48 hours. All samples were plated in duplicate. A reduction in viable cell count by more than 4 log10 was deemed to be bactericidal and was selected for further investigation. Bactericidal compounds were then tested at concentrations of 0.75, 0.5, and 0.25 mM (0.125 mM was also used in some potent compounds) to find the minimum bactericidal concentration. Additional testing at lower concentrations was also done on the eight clinical isolates of European origin.

As shown by the results in Table 1, lauric acid (C12:0), tridecanoic acid (13:0), myristoleic acid (14:1cA9), palmitoleic acid (16:1cA9), linolenic acid (C18:3cΔ9,12,15), monocaprin(C10), and sodium dodecanoate are able to inhibit growth of *Neisseria* *gonorrhoeae* strain NCCP11945 *in vitro* at 1 mM concentration. Other organic acids were also active at this concentration, as noted in Table 1.

**Table 1**

| **Chemical Class** | **Organic Acid** | **Inhibits growth of *Neisseria gonorrhoeae*** |
|---|---|---|
| Saturated Fatty Acids | Caprylic/Octanoic acid 8:0 | No |
| | Capric acid/Decanoic acid 10:0 | No |
| | Undecanoic acid 11:0 | Yes |
| | Lauric acid 12:0 | Yes |
| | Tridecanoic acid 13:0 | Yes |
| | Myristic acid 14:0 | Yes |
| | Pentadecylic acid 15:0 | Yes |
| | Palmitic acid 16:0 | No |
| | Heptadecylic acid 17:0 | No |
| | Stearic acid 18:0 | No |
| Mono-unsaturated Fatty Acids | Myristoleic acid 14:1 cΔ9 | Yes |
| | Palmitoleic acid 16:1 cΔ9 | Yes |
| | Oleic acid 18:1 cΔ9 | Yes |
| | Elaidic acid 18:1 tΔ9 | Yes |
| | Petroselinic acid 18:1 cΔ6 | No |
| | trans-Vaccenic acid 18:1 tΔ11 | No |
| | Cis-Vaccenic acid 18:1 cΔ11 | No |
| | Erucic acid 22:1 cΔ13 | Yes |
| Poly-Unsaturated Fatty Acids | Sorbic acid 6:2 tΔ2,4 | No |
| | Linoleic acid 18:2 cΔ9,12 | Yes |
| | Linolenic acid 18:3 cΔ9,12,15 | Yes |
| | Arachidonic acid 20:4 cΔ5,8,11,14 | Yes |
| Monoglycerides | 1-Octanoyl-rac-Glycerol C8 | Yes |
| | Monocaprin C10 | Yes |
| | Monolaurin C12 | Yes |
| | Monomyristin C14 | Yes |
| Dicarboxylic Acids | Succinic acid | No |
| | Adipic acid | No |
| | Pimelic acid | No |
| | Suberic acid | No |
| | Azelaic acid | No |
| | Sebacic acid | No |
| Ricinoleic Acid | Ricinoleic acid | Yes |
| Esters | Isopropyl myristate | No |
| | Isopropyl palmitate | No |
| Fatty Acid Sodium Salts | Sodium dodecanoate | Yes |
| | Sodium myristate | Yes |

As shown by the results in Table 2, lauric acid (C12:0), tridecanoic acid (C13:0), myristoleic acid (C14:1cA9), palmitoleic acid (C16:1cΔ9), linolenic acid (C18:3cΔ9,12,15), monocaprin(C10), and sodium dodecanoate are able to cause a log reduction in viable *Neisseria gonorrhoeae* strain NCCP11945 bacteria at 1 mM concentration in 2 minutes.

**Table 2**

| **Chemical Class** | **Organic Acid** | **Log reduction of *Neisseria gonorrhoeae*** |
|---|---|---|
| Saturated Fatty Acids | Undecanoic acid 11:0 | 0.2 |
| | Lauric acid 12:0 | >6 |
| | Tridecanoic acid 13:0 | >6 |
| | Myristic acid 14:0 | 1.5 |
| | Pentadecylic acid 15:0 | 0.5 |
| Mono-unsaturated Fatty Acids | Myristoleic acid 14:1 cΔ9 | >6 |
| | Palmitoleic acid 16:1 cΔ9 | >6 |
| | Oleic acid 18:1 cΔ9 | 1.0 |
| | Elaidic acid 18:1 tΔ9 | 0.5 |
| | Erucic acid 22:1 cΔ13 | 0.0 |
| Poly-Unsaturated Fatty Acids | Linoleic acid 18:2 cΔ9,12 | 1.6 |
| | Linolenic acid 18:3 cΔ9,12,15 | >6 |
| | Arachidonic acid 20:4 cΔ5,8,11,14 | Not tested |
| Monoglycerides | 1-Octanoyl-rac-Glycerol C8 | Not tested |
| | Monocaprin C10 | >6 |
| | Monolaurin C12 | 3.4 |
| | Monomyristin C14 | 0.6 |
| Ricinoleic Acid | Ricinoleic acid | 3.5 |
| Fatty Acid Sodium Salts | Sodium dodecanoate | >6 |
| | Sodium myristate | 0.5 |

As shown by the results in Table 3 which presents data on the log reductions of bacterial cell counts, against a panel of nine *N. gonorrhoeae* isolates (strain NCCP11945 and eight isolates from Public Health England), myristoleic acid (C14:1cA9), palmitoleic acid (C16:1cΔ9), linolenic acid (C18:3cΔ9,12,15), and monocaprin (C10) have a minimal bactericidal concentration, defined as the lowest concentration tested to give an average 4 log10 reduction in bacterial count after a two minute exposure, of 0.5 mM. Against a panel of nine N. *gonorrhoeae* isolates, lauric acid (C12:0), tridecanoic acid (C13:0), and sodium dodecanoate have a minimal bactericidal concentration of 0.75 mM.

**Table 3**

| **Chemical Class** | **Organic Acid** | **Minimum bactericidal concentration** |
|---|---|---|
| Saturated Fatty Acids | Lauric acid 12:0 | 0.75 mM |
| | Tridecanoic acid 13:0 | 0.75 mM |
| Mono-unsaturated Fatty Acids | Myristoleic acid 14:1 cΔ9 | 0.5 mM |
| | Palmitoleic acid 16:1 cΔ9 | 0.5 mM |
| Poly-Unsaturated Fatty Acids | Linolenic acid 18:3 cΔ9,12,15 | 0.5 mM |
| Monoglycerides | Monocaprin C10 | 0.5 mM |
| Fatty Acid Sodium Salts | Sodium dodecanoate | 0.75 mM |

### Example 2

### Bovine Corneal Opacity and Permeability (BCOP) test

Bovine eyes were collected from a local slaughter house (ABP, Guildford, Surrey, UK) and were transported in cold saline for testing at the laboratory. Each eye was first examined for signs of damage before use. The test was conducted according to Abdelkader et al., (2012), Int J Pharm 432: 1-10. The exposure time of the test substances was 30 seconds. The selected bactericidal compounds were tested at a concentration of 1 mM in saline. Overall, the concentration of ethanol carry-over from the stock of the organic acids was therefore 1 %. A negative control of saline with 1 % ethanol, an irritant control of 100% acetone, and a strong irritant control of 0.5 M sodium hydroxide, were all tested in each experiment. The test area of the cornea was then washed with 10 ml saline.

Corneal damage to the test area was assessed by fluorescein staining and examined under a cobalt blue filter. Pathological scoring based on opacity, epithelial integrity, and epithelial detachment was done in accordance with previous publication (Vanerp YHM et al., (1990), Toxicology in Vitro 4: 267-269). Each of the seven candidates were tested in triplicate, each test was performed on a separate day from a different batch of eye balls. Corneal histology was done as Abdelkader et al., (2012), Int J Pharm 432: 1-10 by evaluating the damage to the epithelial layer and stroma of the cornea after Haematoxylin and Eosin staining.

As shown by the results in Table 4, the BCOP test predicts that lauric acid (C12:0), tridecanoic acid (13:0), myristoleic acid (14:1cA9), palmitoleic acid (16:1cA9), linolenic acid (C18:3cΔ9,12,15), monocaprin(C10), and sodium dodecanoate will not cause irritation to the human eye. These do not cause significant damage to the surface of the cornea as judged by fluorescein staining and histological examination on three separate occasions in the BCOP test.

**Table 4**

| **Test substance** | **BCOP classification** |
|---|---|
| 0.5 M sodium hydroxide | Strong irritant |
| Acetone | Strong irritant |
| Saline | Non-irritant |
| 1% ethanol | Non-irritant |
| Lauric acid | Non-irritant |
| Myristoleic acid | Non-irritant |
| Palmitoleic acid | Non-irritant |
| Linolenic acid | Non-irritant |
| Monocaprin | Non-irritant |
| Sodium dodecanoate | Non-irritant |
| Tridecanoic acid | Non-irritant |

### Example 3

### Hen's Egg Test - Chorioallantoic Membrane (HET-CAM)

The HET-CAM was performed according to Alany et al., (2006), J Control Release 111: 145-152. Fertilised white leghorn eggs were purchased from Henry Stewart & Co. Ltd (Fakenham, Norfolk, UK) and delivered to Kingston University. All eggs were checked for damage and incubated at 37°C with 60-70 % relative humidity. At day 4 the eggs were removed from their shells and placed in incubation chambers. At day 9, the CAMs were tested. This was done by applying 200 µl of the testing material and recording results at 30, 120, and 300 seconds. At each of these time-points the effect of the substance on the CAM was evaluated by assessing them against the scoring system (Vanerp YHM, et al., (1990), Toxicology in Vitro 4: 267-269).

As shown by the results in Table 5, the (HET-CAM) test predicts that lauric acid (C12:0), tridecanoic acid (13:0), myristoleic acid (14:1cA9), palmitoleic acid (16:1cA9), linolenic acid (C18:3cΔ9,12,15), monocaprin(C10), and sodium dodecanoate will not be irritants to the human eye. Sodium hydroxide was assayed at 0.1M, as a strong irritant control. The organic acids were tested at concentrations of 1 mg/ml, showed no signs of irritation by the HET-CAM test. The HET-CAM test concentration of 1 mg/ml is higher than the bactericidal concentration of 1 mM. This would give a range of 3.6 mM for linolenic acid to 5 mM for lauric acid. As all candidates were totally bactericidal at a concentration of 1 mM this tested concentration was used to demonstrate that these candidates would be totally safe to use.

**Table 5**

| **Test substances** | **Effects** | **Score** | | | **Total Score** |
|---|---|---|---|---|---|
| | | 0.5 min. | 2 min. | 5 min. | |
| Sodium hydroxide | Hyperaemia | X | X | X | 17 - Severe |
| | Haemorrhoage | | | X | |
| | Clotting/coagulation | X | X | X | |
| Acetone | Hyperaemia | X | X | X | 14 - Severe |
| | Haemorrhoage | | | | |
| | Clotting/coagulation | X | X | X | |
| Propylene glycol | Hyperaemia | | X | X | 8 - Moderate |
| | Haemorrhoage | | | | |
| | Clotting/coagulation | | | X | |
| Saline + 10% DMSO | Hyperaemia | | | | 0 - None |
| | Haemorrhoage | | | | |
| | Clotting/coagulation | | | | |
| Lauric acid | Hyperaemia | | | | 0 - None |
| | Haemorrhoage | | | | |
| | Clotting/coagulation | | | | |
| Tridecanoic acid | Hyperaemia | | | | 0 - None |
| | Haemorrhoage | | | | |
| | Clotting/coagulation | | | | |
| Myristoleic acid | Hyperaemia | | | | 0 - None |
| | Haemorrhoage | | | | |
| | Clotting/coagulation | | | | |
| Palmitoleic acid | Hyperaemia | | | | 0 - None |
| | Haemorrhoage | | | | |
| | Clotting/coagulation | | | | |
| Linolenic acid | Hyperaemia | | | | 0 - None |
| | Haemorrhoage | | | | |
| | Clotting/coagulation | | | | |
| Monocaprin | Hyperaemia | | | | 0 - None |
| | Haemorrhoage | | | | |
| | Clotting/coagulation | | | | |

### Example 4

### Red Blood Cell (RBC) lysis assay

Whole bovine blood was obtained from a local slaughter house (ABP, Guildford, Surrey, UK), adding 90 ml blood to 10 ml of 110 mM tri-sodium citrate anticoagulant. RBCs were isolated the same day. The RBCs were washed by three repeats of centrifugation (1,500 x g for 10 minutes) and resuspension in PBSG. Testing was done following European Union Reference Laboratory for alternatives to animal testing (EURL-ECVAM) guidelines (Lewis RW (2010) DB-ALM Protocol No. 99: Red blood cell (RBC) Test System. EURL ECVAM). The organic acids were made up in DMSO and diluted to a DMSO concentration maximum of 10 % (v/v). Samples were shaken at room temperature for 1 hour and then centrifuged at 10,000 rpm for 2 minutes. An aliquot of 750 µl of the supernatant was taken and absorbance at 451 nm tested.

As shown by the results in Table 6, the RBC lysis assay predicts that at their minimum bactericidal concentration lauric acid (C12:0), tridecanoic acid (13:0), myristoleic acid (14:1cA9), monocaprin (C10), and sodium dodecanoate are not irritating.

**Table 6**

| **Fatty Acid** | **H₅₀ (mg/L)** | **H₅₀ (mM)** |
|---|---|---|
| Tridecanoic acid | >1000 | >4.67 |
| Myristoleic acid | 600 - 800 | 2.6 - 3.5 |
| Monocaprin | 400 - 600 | 1.6 - 2.4 |
| Lauric acid | 200 - 400 | 1.0 - 2.0 |
| Sodium dodecanoate | 200 - 400 | 0.9 - 1.8 |
| Palmitoleic acid | 20 - 40 | 0.08 - 0.16 |
| Linolenic acid | 20 - 40 | 0.07 - 0.14 |

### Example 5

### Testing in artificial tear fluid

Artificial tear fluid was made as in Mirejovsky et al., Optometry and Vision Science, (1991), 68: 858-864. The fatty acid candidates were tested in artificial tear fluid to demonstrate that they were still able to kill the bacteria in this environment. *Neisseria gonorrhoeae* strain NCCP11945 was used in these experiments. As the previous log reductions, 107 cells were suspended in GC broth. The cell suspension was centrifuged to remove the culture medium and the bacteria resuspended in the tear fluid. This was then added to the concentrated organic acid candidates. The cells were then counted again after two minute exposure to estimate any reduction due to the exposure to the fatty acid.

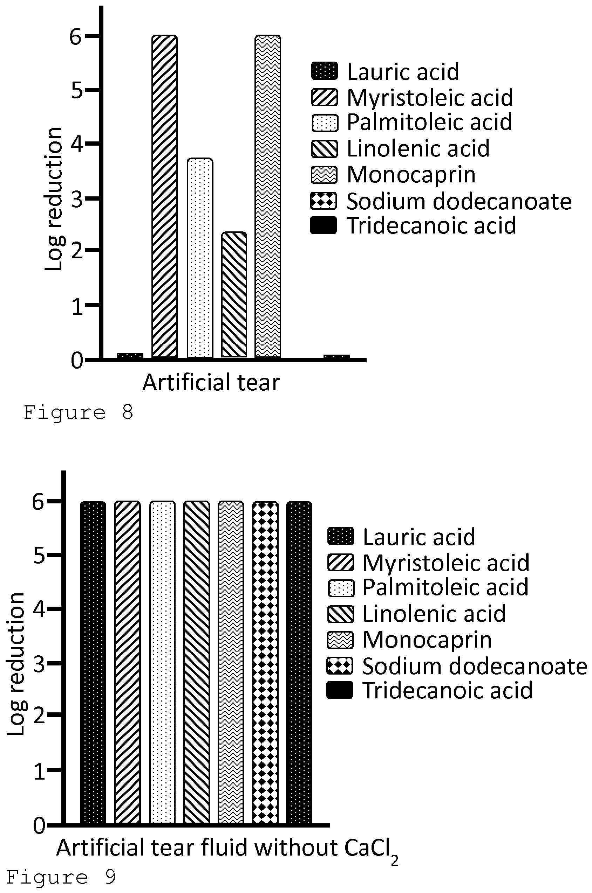

In a first experiment, the artificial tear fluid formulation includes calcium chloride to mimic the calcium in natural tears. As shown by the results in Figure 8, the anti-gonococcal properties of myristoleic acid (C14:1cA9), palmitoleic acid (C16:1cΔ9), linolenic acid (C18:3cΔ9,12,15), and monocaprin(C10) are retained in the artificial tear fluid formulation containing calcium chloride.

In a second experiment, calcium chloride was omitted from the artificial tear fluid formulation. The results in Figure 9 indicate that the anti-gonococcal properties of lauric acid (C12:0), tridecanoic acid (C13:0), and sodium dodecanoate were retained in the artificial tear fluid formulation without calcium chloride.

These results indicate that calcium may reduce or prevent the antimicrobial activity of some of the compounds.

### Example 6

Previously used prophylaxes for ophthalmia neonatorum are applied in the first hour after birth. For use in prophylaxis, the drug must be able to kill the bacterial cells after the bacteria have had sufficient time to infect the human cells. One was tested: monocaprin.

### Cell culture infection model

Primary cornea epithelial cells were obtained from Life Technologies (Paisley, Glasgow, UK) and grown in Keratinocyte serum free medium (Life Technologies), at 37 °C with 5 % CO₂ until 80 % confluent. The cells were then trypsin treated, washed, added to 24 welled plates and incubated for a further 24 hours. The medium was replaced and a fresh P+, Opa+ cells N. *gonorrhoeae* strain NCCP11945 suspension (approximately 107 CFU/ml) was added to the cells to get a multiplicity of infection of 10. All samples were done in duplicate. Monocaprin (1 mM, 0.5 mM, 0.25 mM, and 0.125 mM) was either added directly after the bacteria or after 90 minutes. Non-infection controls showed the morphology of healthy cells, Non-infected Treated controls showed the effect of the drug on the HCECs, Infection controls were positive controls used in log reductions to measure the effect of the drug, and Gentamycin controls compared the action of gentamycin and monocaprin. The total time of the infection was three hours. The cells were washed five times with 1 ml PBS for each well. The wells were treated with warmed 1 % saponin in PBS and incubated for 10 minutes at 37 °C. After this, 900 µl GC broth was added and log reductions calculated as above. The CFUs of the drug exposed wells were compared to the CFU counts of the Infection controls to estimate the log reduction caused by the action of the monocaprin. Comparisons of the log reductions at different drug administration times at given concentrations was analysed in an unpaired t test using GraphPad Prism (version 6.01).

Cell culture wells were alternatively processed for microscopy and contained 13 mm round glass coverslips. After washing in 0.1 M PBS, the cells were fixed in 4 % paraformaldehyde for 20 minutes at room temperature before washing in PBS and blocking with 5 % bovine serum albumin for 30 minutes at room temperature. The wells were washed, each with 1 ml PBS four times. To each well, 200 µl rabbit polyclonal IgG anti-gonococcal antibody (Abcam plc., Cambridge, UK) at a dilution of 1/400 in 0.2% BSA was added and plates incubated at 4 °C overnight. The wells were washed again and a 1/500 dilution of goat polyclonal IgG anti-Rabbit-IgG conjugated to Alexa Fluor 555 and a 1/1000 phalloidin-Fluor 488 (Abcam plc.) in 0.2 % BSA was added for 1 hour and then washed before mounting on a glass slide with Fluoroshield Mounting Medium with 4',6-diamidino-2-phenylindole (DAPI) (Abcam plc.). Slides were viewed using a Nikon Eclipse i80 (Nikon UK Limited) fluorescence microscope and images captured under standard DAPI, TRITC and TRITC filters using NIS-Elements BR 3.0 SP7 (Nikon UK Limited) software. The images were later merged using ImageJ [Schneider CA, Rasband WS, Eliceiri KW: Nature methods, 2012, 9(7):671-675].

### Live/dead staining

The LIVE/DEAD® BacLight™ Bacterial Viability Kit for microscopy (Life Technologies) was used according to manufacturer's instructions. After the course of infection, the wells were washed five times with sterile 0.85 % NaCl to remove non-adhered bacterial cells. Stain was added to each well for 15 minutes at room temperature before washing in 0.85% saline solution. Glass coverslips were mounted on BacLight mounting oil visualised on a Nikon Eclipse i80 (Nikon UK Limited) with standard FITC and TRITC filters using NIS-Elements BR 3.0 SP7 (Nikon UK Limited) software and then merged later using ImageJ.

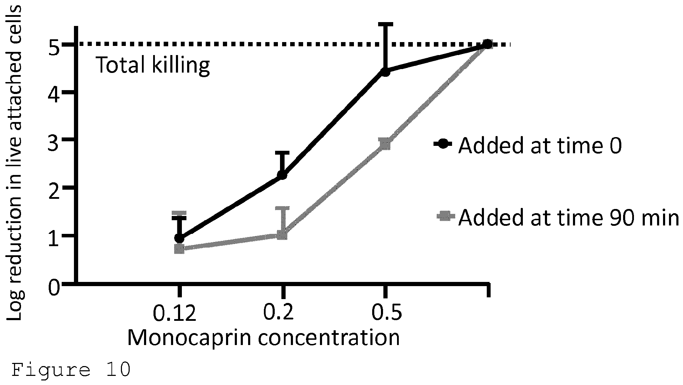

The log reduction results shown in Figure 10 indicate that monocaprin (C10) at 1 mM concentration is able to kill *Neisseria gonorrhoeae* that has infected human epithelial cells for 90 minutes.

## Claims

1. A compound that is a fatty acid or an ester of a fatty acid, or a pharmaceutically acceptable salt or solvate thereof, selected from myristoleic acid C14:1cA9, palmitoleic acid C16:1cA9, and capric acid C10:0 monoglyceride (monocaprin C10), for use in the ocular treatment or prophylaxis of an infection of the eye caused by *Neisseria gonorrhoeae* in a warm-blooded animal.

2. The compound for use according to claim 1, wherein the compound is myristoleic acid C14:1cA9, or capric acid C10:0 monoglyceride (monocaprin C10), or a solvate thereof.

3. The compound for use according to claim 1, wherein the compound is capric acid C10:0 monoglyceride (monocaprin C10).

4. The compound for use according to any of the preceding claims, wherein the compound is in an ophthalmic formulation containing a chelating agent.

5. The compound for use according to any of the preceding claims, wherein the eye infection is in a human.

6. The compound for use according to any of the preceding claims, wherein the eye infection is in a newborn.

7. An ophthalmic formulation comprising a compound as defined in any of claims 1 to 3, and a pharmaceutically acceptable carrier, for use in the ocular treatment or prophylaxis of an infection of the eye caused by *Neisseria gonorrhoeae.*

8. A formulation for use according to claim 7, in the form of an eye drop liquid, an ointment or a hydrogel.

9. A formulation for use according to claim 7 or claim 8, further comprising a chelating agent.

## Patentansprüche

1. Verbindung, die eine Fettsäure oder ein Ester einer Fettsäure oder ein pharmazeutisch verträgliches Salz oder Solvat davon ist, ausgewählt aus Myristoleinsäure C14:1cA9, Palmitoleinsäure C16:1cA9 und Caprinsäure C10:0 Monoglycerid (Monocaprin C10) für die Verwendung in der okularen Behandlung oder Prophylaxe einer durch *Neisseria gonorrhoeae* hervorgerufenen Infektion des Auges bei einem warmblütigen Tier.

2. Verbindung für die Verwendung nach Anspruch 1, wobei die Verbindung Myristoleinsäure C14:1cA9 oder Caprinsäure C10:0 Monoglycerid (Monocaprin C10) oder ein Solvat davon ist.

3. Verbindung für die Verwendung nach Anspruch 1, wobei die Verbindung Caprinsäure C10:0 Monoglycerid (Monocaprin C10) ist.

4. Verbindung nach einem der vorhergehenden Ansprüche, wobei die Verbindung in einer ophthalmischen Formulierung vorliegt, die einen Chelatbildner enthält.

5. Verbindung nach einem der vorhergehenden Ansprüche, wobei die Augeninfektion in einem Menschen vorliegt.

6. Verbindung nach einem der vorhergehenden Ansprüche, wobei die Augeninfektion in einem Neugeborenen vorliegt.

7. Ophthalmische Formulierung, umfassend eine Verbindung, nach einem der Ansprüche 1 bis 3, und einen pharmazeutisch verträglichen Träger zur Verwendung in der okularen Behandlung oder Prophylaxe einer durch *Neisseria gonorrhoeae* hervorgerufenen Infektion des Auges.

8. Formulierung für die Verwendung nach Anspruch 7 in der Form einer Augentropfenflüssigkeit, eines Salbe oder eines Hydrogels.

9. Formulierung für die Verwendung nach Anspruch 7 oder Anspruch 8, ferner umfassend einen Chelatbildner.

## Revendications

1. Composé qui est un acide gras ou un ester d'un acide gras, ou un sel ou un solvate pharmaceutiquement acceptables de celui-ci, choisis parmi l'acide myristoléique C14:1cA9, l'acide palmitoléique C16:1cA9 et l'acide caprique C10:0 monoglycéride (monocaprine C10), pour une utilisation dans le traitement ou la prophylaxie oculaire d'une infection de l'œil entraînée par *Neisseria gonorrhoeae* chez un animal à sang chaud.

2. Composé pour une utilisation selon la revendication 1, où le composé est l'acide myristoléique C14:1cA9 ou l'acide caprique C10:0 monoglycéride (monocaprine C10), ou un solvate de celui-ci.

3. Composé pour une utilisation selon la revendication 1, où le composé est l'acide caprique C10:0 monoglycéride (monocaprine C10).

4. Composé pour une utilisation selon l'une quelconque des revendications précédentes, où le composé est dans une formulation ophtalmique contenant un agent chélatant.

5. Composé pour une utilisation selon l'une quelconque des revendications précédentes, où l'infection de l'œil est chez un humain.

6. Composé pour une utilisation selon l'une quelconque des revendications précédentes, où l'infection de l'œil est chez un nouveau-né.

7. Formulation ophtalmique comprenant un composé selon l'une quelconque des revendications 1 à 3 et un véhicule pharmaceutiquement acceptable, pour une utilisation dans le traitement ou la prophylaxie oculaire d'une infection de l'œil entraînée par *Neisseria gonorrhoeae.*

8. Formulation pour une utilisation selon la revendication 7, sous la forme d'un liquide de gouttes ophtalmiques, d'un onguent ou d'un hydrogel.

9. Formulation pour une utilisation selon la revendication 7 ou la revendication 8, comprenant en outre un agent chélatant.
